# EUROPEAN PATENT APPLICATION

(11) **EP 1 806 159 A1**
(43) Date of publication of application: **11.07.2007**
(21) Application number: 05782268.6
(22) Date of filing: 07.09.2005
(51) Int. Cl.: A61M 21/02, F24F 11/02, H04Q 9/00

(54) **ENVIRONMENT ADJUSTMENT SYSTEM, ENVIRONMENT ADJUSTMENT METHOD, AIR CONDITIONER, AND REMOTE CONTROLLER**

(30) Priority: 30.09.2004 JP 2004287887
(71) Applicant: DAIKIN INDUSTRIES, LTD., Osaka-shi, Osaka 530-8323 (JP)
(72) Inventor: ISHIWATA, Takayuki Shiga Plant DAIKIN Ind. Ltd., Kusatsu-shi Shiga 5258526 (JP); KINO, Shoko Shiga Plant DAIKIN INDUSTRIES, LTD., Kusatsu-shi Shiga 5258526 (JP); ARAI, Jun-ichiro Shiga Plant DAIKIN INDUSTRIES LTD, Kusatsu-shi Shiga 5258526 (JP)
(74) Representative: Goddar, Heinz J.
(86) International application number: PCT/JP2005/016445
(87) International publication number: WO 2006/038419

(57) **Abstract**

The present invention relates to techniques for adjusting environment, and has an object to obtain a favorable sleeping state while looking at an image projected on a display such as a television. To attain the object, an environment adjustment system mainly includes an air conditioner (1) and a display (2). The air conditioner (1) includes a temperature adjustment control part (11), a transmission part (12), and a temperature adjustment part (13). The display (2) includes a projection control part (21), a reception part (23), and a projection part (24). The temperature adjustment control part (11) causes the transmission part (12) to transmit a control instruction (S21) to the display (2). The control instruction (S21) causes the display (2) to control the color tone of an image. The reception part (23) receives the control instruction (S21) transmitted from the air conditioner (1), and the projection control part (21) causes the projection part (24) to display an image with a color tone having a calming effect based on the control instruction (S21). After that, the temperature adjustment control part (11) causes the temperature adjustment part (13) to lower ambient temperature.

## Description

### Technical Field

The present invention relates to techniques for adjusting environment, and can be applied to sleep control techniques, for example.

### Background Art

Human beings adjust their biological rhythms with light illuminance or the like. A biological rhythm is a life-related rhythm such as, for example, wakeup time and bedtime, and sleeping hours.

In this day and age, there are many factors that disturb the biological rhythm. For example, the biological rhythm tends to be disturbed after the person stays up late. Therefore, techniques for changing the disturbed biological rhythm back to normal have conventionally been developed. For example, a patent document 1 discloses a technique for adjusting ambient temperature based on measured illuminance and ambient temperature. A patent document 2 discloses a technique for adjusting the biological rhythm by controlling light illuminance.

The following documents disclose techniques pertinent to the present invention.
A patent document 3 discloses a technique for controlling ambient temperature to obtain a favorable sleeping state. Patent documents 4 and 5 disclose remote control techniques for unified management of a plurality of devices.

Patent document 1: Japanese Patent Application Laid-Open No. 8-193738
Patent document 2: Japanese Patent Application Laid-Open No. 2000-252084
Patent document 3: Japanese Patent No. 2987981
Patent document 4: Japanese Patent Application Laid-Open No. 2003-125465
Patent document 5: Japanese Patent Application Laid-Open No. 2003-018672

### Disclosure of Invention

### Problems to be Solved by the Invention

With the spread of television, many people watch TV installed in their bedrooms before going to bed. This results in delayed bedtime, causing their biological rhythms to be disturbed.

When the technique disclosed in the patent document 2 is applied to such case, light from a television may hinder its effects.

The prevent invention has been made in view of the circumstances, and has an object to obtain a favorable sleeping state while looking at an image projected on a display such as a television.

### Means for Solving the Problems

In a first aspect of an environment adjustment system of the present invention, an environment adjustment system includes: a projection part (24) for displaying or projecting an image with a variable color tone; a temperature adjustment part (13) for adjusting ambient temperature; and a control part (11, 21) for controlling the projection part and the temperature adjustment part respectively, with variations in the color tone of the image and the adjustment to the ambient temperature associated with each other.

In a second aspect of the environment adjustment system of the present invention, in the environment adjustment system according to the first aspect, the projection part (24) changes the color tone to one having a calming effect, and then the temperature adjustment part (13) lowers the ambient temperature.

In a third aspect of the environment adjustment system of the present invention, the environment adjustment system according to the second aspect further includes a sleep sensor for detecting the onset of sleep, wherein the control part (11) controls the temperature adjustment part (13) to lower the ambient temperature from the onset of sleep detected by the sleep sensor.

In a fourth aspect of the environment adjustment system of the present invention, in the environment adjustment system according to the second or third aspect, a color temperature set for the projection part (24) decreases upon changing the color tone to the one having a calming effect.

In a fifth aspect of the environment adjustment system of the present invention, in the environment adjustment system according to any one of the second through fourth aspects, the luminance of the image decreases upon changing the color tone to the one having a calming effect.

In a sixth aspect of the environment adjustment system of the present invention, the environment adjustment system according to any one of the second through fifth aspects further includes a sound generation part (4) for generating sound, wherein the volume of the sound decreases with the change in the color tone to the one having a calming effect.

In a seventh aspect of the environment adjustment system of the present invention, in the environment adjustment system according to the first aspect, the temperature adjustment part (13) raises the ambient temperature, and then the projection part (24) changes the color tone to one having a stimulating effect.

In an eighth aspect of the environment adjustment system of the present invention, the environment adjustment system according to the seventh aspect further includes an arousal sensor for detecting the onset of arousal, wherein the control part (11) controls the projection part (24) to change the color tone of the image to one having a stimulating effect from the onset of arousal detected by the arousal sensor.

In a ninth aspect of the environment adjustment system of the present invention, in the environment adjustment system according to the seventh or eighth aspect, a color temperature set for the projection part (24) increases upon changing the color tone to the one having a stimulating effect.

In a tenth aspect of the environment adjustment system of the present invention, in the environment adjustment system according to any one of the seventh through ninth aspects, the luminance of the image increases upon changing the color tone to the one having a stimulating effect.

In an eleventh aspect of the environment adjustment system of the present invention, the environment adjustment system according to any one of the seventh through tenth aspects further includes a sound generation part (4) for generating sound,
wherein the volume of the sound increases with the change in the color tone to the one having a stimulating effect.

In a twelfth aspect of the environment adjustment system of the present invention, in the environment adjustment system according to any one of the second through eleventh aspects, the control part includes a temperature adjustment control part (11) for controlling an operation of the temperature adjustment part (13), and a projection control part (21) for controlling an operation of the projection part (24), and the environment adjustment system is provided with an air conditioner (1) including the temperature adjustment part and the temperature adjustment control part, and a display (2) including the projection part and the projection control part.

In a thirteenth aspect of the environment adjustment system of the present invention, in the environment adjustment system according to the sixth or eleventh aspect, the control part includes a temperature adjustment control part (11) for controlling an operation of the temperature adjustment part (13), a projection control part (21) for controlling an operation of the projection part (24), and a sound control part (21) for controlling an operation of the sound generation part (4), and the environment adjustment system is provided with an air conditioner (1) including the temperature adjustment part and the temperature adjustment control part, and a display (2) including the projection part, the projection control part, and the sound control part.

In a fourteenth aspect of the environment adjustment system of the present invention, in the environment adjustment system according to the twelfth or thirteenth aspect, the air conditioner (1) further includes a transmission part (12) for transmitting a first control instruction (S21), the display (2) further includes a first reception part (23) for receiving the first control instruction, the temperature adjustment control part (11) causes the transmission part to transmit the first control instruction to the first reception part, and the projection control part (21) causes the projection part to display or project the image with a change in the color tone based on the first control instruction received by the first reception part.

In a fifteenth aspect of the environment adjustment system of the present invention, the environment adjustment system according to the fourteenth aspect further includes a remote controller (3) for transmitting a second control instruction (S11),
wherein the air conditioner (1) further includes a second reception part (14) for receiving the second control instruction, and the temperature adjustment control part (11) causes the temperature adjustment part (13) to control the ambient temperature based on the second control instruction received by the second reception part, and causes the transmission part (12) to transmit the first control instruction (S21) to the first reception part (23) based on the second control instruction.

In a sixteenth aspect of the environment adjustment system of the present invention, the environment adjustment system according to the twelfth or thirteenth aspect further includes a remote controller (3) for transmitting a first control instruction (S21), wherein the air conditioner (1) further includes a transmission part (12) for transmitting a second control instruction (S201), the display (2) further includes a first reception part (23) for receiving the first control instruction, the temperature adjustment control part (11) causes the transmission part (12) to transmit the second control instruction to the remote controller, the remote controller transmits the first control instruction to the first reception part based on the second control instruction, and the projection control part (21) causes the projection part to display or project the image with a change in the color tone based on the first control instruction received by the first reception part.

In a seventeenth aspect of the environment adjustment system of the present invention, the environment adjustment system according to the twelfth or thirteenth aspect further includes a remote controller (3) including: a transmission part (32) for transmitting a first control instruction (S21) instructing control of the projection part (24) and a second control instruction (S11) instructing control of the temperature adjustment part (13); and a transmission control part (31) for controlling the transmission part,
wherein the display (2) further includes a first reception part (23) for receiving the first control instruction, the air conditioner (1) further includes a second reception part (14) for receiving the second control instruction, the transmission control part causes the transmission part to transmit the first control instruction to the first reception part and the second control instruction to the second reception part, the projection control part (21) causes the projection part (24) to display or project the image with a change in the color tone based on the first control instruction received by the first reception part, and the temperature adjustment control part (11) causes the temperature adjustment part (13) to control the ambient temperature based on the second control instruction received by the second reception part.

In an eighteenth aspect of the environment adjustment system of the present invention, in the environment adjustment system according to the seventeenth aspect, a third control instruction (S1) instructing control of the air conditioner (1) is input to the transmission control part (31), and the transmission control part causes the transmission part (32) to transmit the second control instruction (S11) based on the third control instruction to the second reception part (14), and causes the transmission part to transmit the first control instruction (S21) based on the third control instruction to the first reception part (23).

In a nineteenth aspect of the environment adjustment system of the present invention, in the environment adjustment system according to the seventeenth aspect, a third control instruction (S2) instructing control of the display (2) is input to the transmission control part (31), and the transmission control part causes the transmission part (32) to transmit the first control instruction (S21) based on the third control instruction to the first reception part (23), and causes the transmission part to transmit the second control instruction (S11) based on the third control instruction to the second reception part (14).

In a first aspect of an environment adjustment method of the present invention, the method includes: a projection step of changing a color tone of an image displayed or projected on a display (2) to one having a calming effect; and a temperature adjustment step of lowering ambient temperature after the projection step.

In a second aspect of the environment adjustment method of the present invention, in the environment adjustment method according to the first aspect, a color temperature of the color tone is lowered upon changing the color tone to the one having a calming effect in the projection step.

In a third aspect of the environment adjustment method of the present invention, in the environment adjustment method according to the first or second aspect, a color temperature of the color tone is lowered upon changing the color tone to the one having a calming effect in the projection step.

In a fourth aspect of the environment adjustment method of the present invention, in the environment adjustment method according to any one of the first through third aspects, the luminance of the image is decreased upon changing the color tone to the one having a calming effect in the projection step.

In a fifth aspect of the environment adjustment method of the present invention, in the environment adjustment method according to any one of the first through fourth aspects, a sound generation part (4) generates sound in parallel with the display or projection of the image on the display (2), and the volume of the sound is decreased with the change in the color tone to the one having a calming effect in the projection step.

In a sixth aspect of the environment adjustment method of the present invention, the method includes: a temperature adjustment step of raising ambient temperature; and a projection step of changing a color tone of an image displayed or projected on a display (2) to one having a stimulating effect after the temperature adjustment step.

In a seventh aspect of the environment adjustment method of the present invention, in the environment adjustment method according to the sixth aspect, the color tone is changed to the one having a simulating effect from the onset of arousal in the projection step.

In an eighth aspect of the environment adjustment method of the present invention, in the environment adjustment method according to the sixth or seventh aspect, a color temperature of the color tone is raised upon changing the color tone to the one having a stimulating effect in the projection step.

In a ninth aspect of the environment adjustment method of the present invention, in the environment adjustment method according to any one of the sixth through eighth aspects, the luminance of the image is increased upon changing the color tone to the one having a stimulating effect in the projection step.

In a tenth aspect of the environment adjustment method of the present invention, in the environment adjustment method according to any one of the sixth through ninth aspects, a sound generation part (4) generates sound in parallel with the display or projection of the image on the display (2), and the volume of the sound is increased with the change in the color tone to the one having a stimulating effect in the projection step.

In an eleventh aspect of the environment adjustment method of the present invention, the method is for an environment adjustment system, the system including: an air conditioner (1) for executing the temperature adjustment step according to any one of the first through tenth aspects of the environment adjustment system; and a display (2) for executing the projection step according to any one of the claims, the air conditioner including a transmission part (12) for transmitting a first control instruction (S21), and the display including a first reception part (23), the first reception part receiving the first control instruction, the method including: a transmission step of causing the transmission part to transmit the first control instruction to the first reception part; and a step of executing the projection step based on the first control instruction after receiving the first control instruction by the first reception part.

In a twelfth aspect of the environment adjustment method of the present invention, the environment adjustment method according to the eleventh aspect further includes a remote controller (3) for transmitting a second control instruction (S11), the air conditioner (1) further including a second reception part (14) for receiving the second control instruction, the method executing, before the transmission step,: a step of causing the remote controller to transmit the second control instruction; and a step of executing the temperature adjustment step based on the second control instruction after receiving the second control instruction by the second reception part, and generating the first control instruction (S21) based on the second control instruction in the air conditioner (1).

In a thirteenth aspect of the environment adjustment method of the present invention, the method is for an environment adjustment system, the system including: an air conditioner (1) for executing the temperature adjustment step according to any one of the first through tenth aspects of the environment adjustment system; and a display (2) for executing the projection step according to any one of the claims, the air conditioner including a transmission part (12); and a remote controller (3) for transmitting a first control instruction (S21), the air conditioner (1) including a transmission part (12) for transmitting a second control instruction (S201), and the display (2) including a first reception part (23) for receiving the first control instruction, the method including: a step of causing the transmission part to transmit the second control instruction to the remote controller; a step of causing the remote controller having received the second control instruction to transmit the first control instruction to the first reception part based on the second control instruction; and a step of executing the projection step based on the first control instruction after receiving the first control instruction by the first reception part.

In a fourteenth aspect of the environment adjustment method of the present invention, the method is for an environment adjustment system, the system including: an air conditioner (1) for executing the temperature adjustment step according to any one of the first through tenth aspects of the environment adjustment system; a display (2) for executing the projection step according to any one of the claims; and a remote controller (3), the display (2) further including a first reception part (23), the first reception part receiving a first control instruction (S21), the air conditioner (1) further including a second reception part (14) for receiving a second control instruction (S11), and the remote controller including a transmission part (32) for transmitting the first and second control instructions, the method including the steps of: (a) causing the transmission part to transmit the first control instruction (S21) to the first reception part (23) and the second control instruction (S11) to the second reception part (14); (b) executing the projection step based on the first control instruction after receiving the first control instruction by the first reception part; and (c) executing the temperature adjustment step based on the second control instruction after receiving the second control instruction by the second reception part.

In a fifteenth aspect of the environment adjustment method of the present invention, the method executes, before the step (a), a step of inputting a third control instruction (S1) instructing execution of the temperature adjustment step to the remote controller (3), wherein in the step (a), the transmission part (32) transmits the second control instruction (S11) based on the third control instruction to the second reception part (14), and the transmission part transmits the first control instruction (S21) based on the third control instruction to the first reception part (23).

In a sixteenth aspect of the environment adjustment method of the present invention, the method executes, before the step (a), a step of inputting a third control instruction (S2) instructing execution of the projection step to the remote controller (3),
wherein in the step (a), the transmission part (32) transmits the first control instruction (S21) based on the third control instruction to the first reception part (23), and the transmission part transmits the second control instruction (S 11) based on the third control instruction to the second reception part (14).

An air conditioner according to the present invention includes: a transmission part (12) for transmitting a first control instruction (S21) to a display (2) for displaying or projecting an image with a variable color tone; a temperature adjustment part (13) for adjusting ambient temperature; and a temperature adjustment control part (11) for controlling the temperature adjustment part, the temperature adjustment control part causing the transmission part to transmit the first control instruction to the display, and the first control instruction causing the display to control the color tone of the image in association with the temperature adjustment control part causing the temperature adjustment part to adjust the ambient temperature.

A remote controller according to the present invention includes: a transmission part (32); and a transmission control part (31) for controlling the transmission part, the transmission part being capable of transmitting a first control instruction (S21) to a display (2) for displaying or projecting an image with a variable color tone, and of transmitting a second control instruction (S11) to an air conditioner (1), the transmission control part causing the transmission part to transmit the first control instruction (S21) to the display and the second control instruction (S11) to the air conditioner, the first control instruction causing the display to control a color tone of the image, the second control instruction causing the air conditioner to adjust ambient temperature, and variations in the color tone of the image and the adjustment to the ambient temperature are associated with each other.

### Effects of the Invention

According to the first aspect of the environment adjustment system, the air conditioner or the remote controller of the present invention, sleep/arousal control by temperature and sleep/arousal control by color tone may be associated with each other. Further, with a non-monochrome image being displayed, a person can sleep or wake up while enjoying information included in the image.

According to the second aspect of the environment adjustment system or the first aspect of the environment adjustment method of the present invention, the person looking at the image feels a color having a calming effect with the change of color tone of the image displayed or the like to one having a calming effect, and is encouraged to fall asleep. And with the subsequent decrease in ambient temperature, the body temperature decreases during sleep. Accordingly, the person is smoothly induced to sleep.

According to the third aspect of the environment adjustment system or the second aspect of the environment adjustment method of the present invention, the ambient temperature decreases from the onset of sleep. Accordingly, the person does not feel uncomfortable.

According to the fourth aspect of the environment adjustment system or the third aspect of the environment adjustment method of the present invention, a calming effect is provided with the color tone of a lowered color temperature. Accordingly, the person looking at the image displayed or the like with the color tone is encouraged to fall asleep.

According to the fifth aspect of the environment adjustment system or the fourth aspect of the environment adjustment method of the present invention, the surroundings grow dark. Accordingly, the person looking at the image is encouraged to fall asleep.

According to the sixth aspect of the environment adjustment system or the fifth aspect of the environment adjustment method of the present invention, the surroundings go quiet. Accordingly, the person listening to the sound is encouraged to fall asleep.

According to the seventh aspect of the environment adjustment system or the sixth aspect of the environment adjustment method of the present invention, the increase in ambient temperature causes the body temperature to increase during sleep, inducing the sleeping person to arousal. And the person looking at the image subsequently displayed or the like after arousal feels a color having a stimulating effect with the change of color tone to one having a stimulating effect, and feels less drowsy. Accordingly, the person is smoothly induced to arousal.

According to the eighth aspect of the environment adjustment system or the seventh aspect of the environment adjustment method of the present invention, the color tone of the image changes to the one having a stimulating effect from the onset of arousal. Accordingly, the person feels less uncomfortable resulting from accelerated arousal.

According to the ninth aspect of the environment adjustment system or the eighth aspect of the environment adjustment method of the present invention, a stimulating effect is provided with the color tone of a raised color temperature. Accordingly, the person looking at the image displayed or the like with the color tone feels less drowsy.

According to the tenth aspect of the environment adjustment system or the ninth aspect of the environment adjustment method of the present invention, the surroundings grow light. Accordingly, the person looking at the image feels less drowsy.

According to the eleventh aspect of the environment adjustment system or the tenth aspect of the environment adjustment method of the present invention, the surroundings get loud. Accordingly, the person listening to the sound feel less drowsy.

According to the twelfth or thirteenth aspect of the environment adjustment system of the present invention, the environment adjustment system may be made of an air conditioner and a display.

According to the fourteenth aspect of the environment adjustment system or the eleventh aspect of the environment adjustment method of the present invention, each of the air conditioner and the display is provided separately, and the air conditioner remotely controls the display based on the first control instruction. Accordingly, the control of the air conditioner and the control of the display may be associated with each other.

According to the fifteenth aspect of the environment adjustment system or the twelfth aspect of the environment adjustment method of the present invention, only the input of the control instruction for the air conditioner to the remote controller allows the display to be automatically controlled.

According to the sixteenth aspect of the environment adjustment system or the thirteenth aspect of the environment adjustment method of the present invention, the air conditioner automatically controls the display via the remote controller.

According to the seventeenth aspect of the environment adjustment system or the fourteenth aspect of the environment adjustment method of the present invention, the remote controller is capable of managing the air conditioner and the display in a unified manner. Accordingly, the control of the air conditioner and the control of the display may easily be associated with each other.

According to the eighteenth aspect of the environment adjustment system or the fifteenth aspect of the environment adjustment method of the present invention, only the input of the control instruction for the air conditioner to the remote controller allows the display to be automatically controlled.

According to the nineteenth aspect of the environment adjustment system or the sixteenth aspect of the environment adjustment method of the present invention, only the input of the control instruction for the display to the remote controller allows the air conditioner to be automatically controlled.

These and other objects, features, aspects and advantages of the present invention will become more apparent from the following detailed description when taken in conjunction with the accompanying drawings.

### Brief Description of Drawings

Fig. 1 is a schematic block diagram of an environment adjustment system described in a first preferred embodiment.
Fig. 2 is a schematic block diagram of an environment adjustment system described in the first preferred embodiment.
Fig. 3 is a schematic block diagram of an environment adjustment system described in the first preferred embodiment.
Fig. 4 is a schematic block diagram of an environment adjustment system described in a second preferred embodiment.
Fig. 5 is a schematic block diagram of an environment adjustment system described in the second preferred embodiment.
Fig. 6 is a schematic block diagram of an environment adjustment system described in a second preferred embodiment.
Fig. 7 is a schematic block diagram of an environment adjustment system described in a third preferred embodiment.
Fig. 8 is a schematic block diagram of an environment adjustment system described in the third preferred embodiment.
Fig. 9 is a schematic block diagram of a usage pattern of the environment adjustment system.

### Best Modes for Carrying Out the Invention

### First Preferred Embodiment

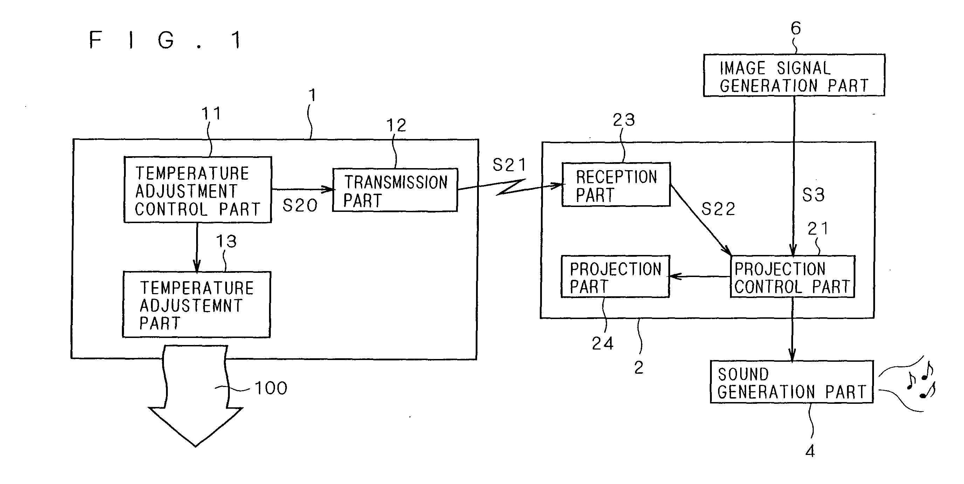

Fig. 1 is a schematic block diagram of an environment adjustment system according to this preferred embodiment. The system mainly includes an air conditioner 1 and a display 2.

The air conditioner 1 includes a temperature adjustment control part 11. a transmission part 12, and a temperature adjustment part 13. The temperature adjustment control part 11 controls the transmission part 12 and the temperature adjustment part 13 as set out below.

The transmission part 12 is caused to input a control instruction S20 to transmit a control instruction S21 to the display 2 based on the control instruction S20. The control instruction S21 causes the display 2 to control the color tone or the like of an image. The image shall mean a still image or a moving image, and does not include a monochrome screen.

The temperature adjustment part 13 is caused to adjust ambient temperature. The temperature adjustment part 13 discharges temperature adjusted air 100 into the environment of a prescribed position, e.g. an environment in which the air conditioner 1 is installed, thereby adjusting the temperature of the environment, namely, ambient temperature.

The display 2 includes a projection control part 21, a reception part 23, and a projection part 24. In Fig. 1, the display 2 is connected to a sound generation part 4 and an image signal generation part 6. While being externally attached to the display 2, the sound generation part 4 and the image signal generation part 6 may be incorporated in the display 2.

The reception part 23 receives the control instruction S21 transmitted from the air conditioner 1, and inputs the same as a control instruction S22 to the projection control part 21.

The projection control part 21 controls the projection part 24, the sound generation part 4, and the image signal generation part 6 as set out below.

The image signal generation part 6 is caused to output an image signal S3. The image signal generation part 6 is, for example, a tuner, a video recorder, a DVD (Digital Versatile Disk) recorder, a terminal or the like. The tuner selects and receives a channel desired by the projection control part 21 from VHF (Very High Frequency)- and UHF (Ultra High Frequency)-band television signals, and outputs the same as the image signal S3 to the projection control part 21. The video recorder or DVD recorder reads images from a video or a DVD, and outputs the same as the image signal S3 to the projection control part 21. The terminal captures images from a network, for example, and outputs the same as the image signal S3 to the projection control part 21.

The projection part 24 is caused to display an image on a screen of the display 2 or projects an image on a screen installed in a different position from the display 2 based on the image signal S3 obtained from the image signal generation part 6. The image signal S3 may be supplied to the projection part 24 directly from the image signal generation part 6 without going through the projection control part 21.

The image displayed or the like by the projection part 24 is subjected to color tone control based on the control instruction S22, namely, based on the control instruction S21. More specifically, the color tone is controlled to have a calming effect for bedtime, and a stimulating effect for wakeup time.

The sound generation part 4 is caused to generate sound in parallel with the display or projection of the image by the projection part 24. The sound generation part 4 is a speaker, for example. More specifically, a sound signal generated by a sound signal generation part incorporated in the image signal generation part may be supplied together with the image signal to the projection control part 21, causing the sound generation part 4 to generate sound based on the sound signal. At this time, the sound generation part 4 may be controlled by a sound control part provided separately from the projection control part 21.

The control of the temperature adjustment part 13 in the air conditioner 1 and the control of the display 2 based on the control instruction S21, namely, the control of the color tone of an image, are linked with each other. The environment adjustment system may therefore be understood as follows: namely, the environment adjustment system includes the projection part 24, the temperature adjustment part 13, and a control part. The projection part 24 displays or projects an image with a variable color tone. The temperature adjustment part 13 adjusts ambient temperature. The control part includes the temperature adjustment control part 11 and the projection control part 21 that control the temperature adjustment part 13 and the projection part 24, respectively, with variations in color tone of the image and ambient temperature adjustment associated with each other.

In this environment adjustment system, sleep/arousal control by temperature and sleep/arousal control by color tone may be associated with each other. Further, with a non-monochrome image being displayed, a person can sleep or wake up while enjoying information included in the image.

The control technique by the environment adjustment system will now be described for bedtime (sleep) and for wakeup time (arousal) independently.

For bedtime, the projection part 24 changes the color tone to one having a calming effect, and then the temperature adjustment part 13 lowers ambient temperature.

More specifically, the projection control part 21 first causes the projection part 24 to display or project an image with a color tone having a calming effect based on the control instruction S22, namely, based on the control instruction S21, in the display 2.

The color tone having a calming effect is obtained by changing the white color temperature to not more than 6000 K. A person looking at the image displayed or the like with this color tone is encouraged to fall asleep.

In addition, the luminance of the image may be decreased in parallel with obtaining the color tone having a calming effect. As the surroundings grow dark, the person looking at the image is further encouraged to fall asleep.

Further, the volume of sound generated by the sound generation part 4 may be decreased in parallel with obtaining the color tone having a calming effect. As the surroundings go quiet, the person listening to the sound is further encouraged to fall asleep.

After the above control, the temperature adjustment control part 11 causes the temperature adjustment part 13 to lower ambient temperature in the air conditioner 1. In this case, it is preferable for the ambient temperature to be lowered from the onset of sleep, so as not to make the person uncomfortable with the feeling of being chilly or the like. The onset of sleep is detected by a sleep sensor, for example.

With such control technique, the person looking at the image feels a color having a calming effect with the change of color tone of the image displayed or the like to one having a calming effect, and is encouraged to fall asleep. And with the subsequent decrease in ambient temperature, the body temperature decreases during sleep. Accordingly, the person is smoothly induced to sleep.

As for wakeup time, the temperature adjustment part 13 raises ambient temperature, and then the projection part 24 changes the color tone to one having a stimulating effect.

More specifically, the temperature adjustment control part 11 first causes the temperature adjustment part 13 to raise ambient temperature in the air conditioner 1.

Then, the projection control part 21 causes the projection part 24 to display or project an image with a color tone having a stimulating effect based on the control instruction S22, namely, based on the control instruction S21, in the display 2. It is preferable for the color tone to have a stimulating effect from the onset of arousal, so as not to make the person uncomfortable with accelerated arousal or the like. The onset of arousal is detected by an arousal sensor, for example.

The color tone having a stimulating effect is obtained by changing the white color temperature to not less than 10000 K. A person looking at the image displayed or the like with this color tone feels less drowsy.

In addition, the luminance of the image may be increased in parallel with obtaining the color tone having a stimulating effect. Accordingly, as the surroundings grow light, the person looking at the image feels less drowsy.

Further, the volume of sound generated by the sound generation part 4 may be increased in parallel with obtaining the color tone having a stimulating effect. As the surroundings get loud, the person listening to the sound feels less drowsy.

With such control technique, the increase in ambient temperature causes the body temperature to increase during sleep, inducing the sleeping person to arousal. And the person looking at the image subsequently displayed or the like after arousal feels a color having a stimulating effect with the change of color tone to one having a stimulating effect, and feels less drowsy. Accordingly, the person is smoothly induced to arousal.

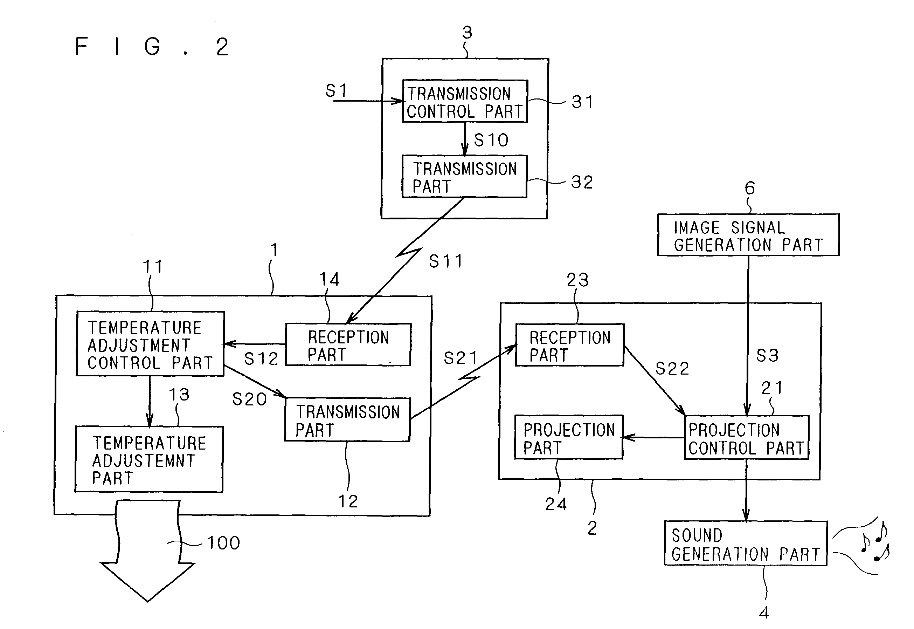

Fig. 2 is a schematic block diagram of the environment adjustment system including a remote controller 3 that communicates with the air conditioner 1. In an aspect shown in Fig. 2, the air conditioner 1 may control ambient temperature based on an externally input control instruction S11. The same elements as those of Fig. 1 have the same reference numerals in Fig. 2.

The remote controller 3 includes a transmission control part 31 and a transmission part 32. A control instruction S1 for the air conditioner 1 is input to the remote controller 3. The control instruction S1 includes instructions about ambient temperature to be adjusted by the temperature adjustment part 13, and about starting time of the control.

The transmission control part 31 controls the transmission part 32. More specifically, a control instruction S10 is supplied to the transmission part 32 based on the control instruction S1, and the transmission part 32 is caused to transmit the control instruction S 11 to the air conditioner 1 based on the control instruction S10. The control instruction S 11 causes the air conditioner 1 to control ambient temperature based on the control instruction S 1.

The air conditioner 1 further includes a reception part 14. The reception part 14 receives the control instruction S11 transmitted from the remote controller 3, and inputs the same as a control instruction S12 to the temperature adjustment control part 11.

The temperature adjustment control part 11 causes the temperature adjustment part 13 to control ambient temperature based on the control instruction S12, namely, based on the control instruction S11. Further, the control instruction S20 that causes the transmission part 12 to transmit the control instruction S21 is generated based on the control instruction S12, namely, based on the control instruction S11. By inputting the control instruction S20, the transmission part 12 is caused to transmit the control instruction S21 to the display 2. The control instruction S21 causes the display 2 to control the color tone or the like of an image, as described above.

The display 2 receives the control instruction S21 by the reception part 23 and, as described above, controls the color tone of an image, and further the luminance of the image and the volume of sound.

Again in this case, the control of the display 2 based on the control instruction S21 and the control of the temperature adjustment part 13 in the air conditioner are linked with each other as described above.

With such control technique, only the input of the control instruction S 1 for the air conditioner 1 to the remote controller 3 allows the display 2 to be automatically controlled.

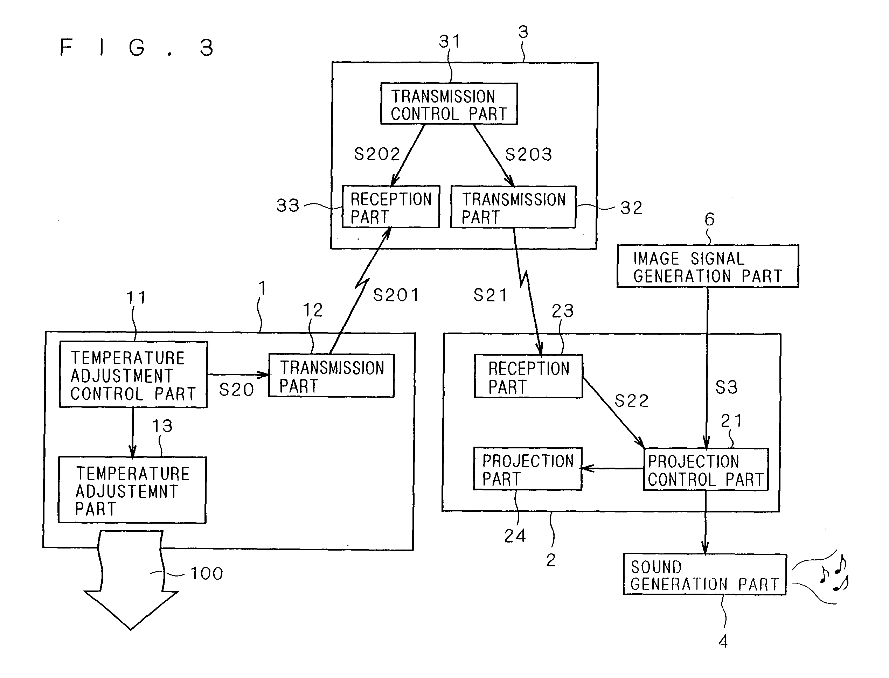

Fig. 3 is a schematic block diagram of another example of the environment adjustment system. In an aspect shown in Fig. 3, the air conditioner 1 may cause the display 2 to control the color tone of an image via the remote controller 3. The same elements as those of Fig. 1 have the same reference numerals in Fig. 3.

In the air conditioner 1, the temperature adjustment control part 11 causes the transmission part 12 to transmit a control instruction S201 to the remote controller 3. The control instruction S201 causes the display 2 to control the color tone of an image via the remote controller 3.

The remote controller 3 includes the transmission control part 31, the transmission part 32, and a reception part 33.

The reception part 33 receives the control instruction S201 transmitted from the air conditioner 1, and inputs the same as a control instruction S202 to the transmission control part 31.

The transmission control part 31 supplies a control instruction S203 to the transmission part 32 based on the control instruction S202, causing the transmission part 32 to transmit the control instruction S21 to the display 2.

The display 2 receives the control instruction S21 by the reception part 23 and, as described above, controls the color tone of an image, and further the luminance of the image and the volume of sound.

Again in this case, the control of the display 2 based on the control instruction S21 and the control of the temperature adjustment part 13 in the air conditioner are linked with each other as described above.

With such control technique, the air conditioner 1 is capable of automatically controlling the display 2 via the remote controller 3.

In any of the control techniques described above, the temperature adjustment control part 11 may cause the transmission part 12 or the remote controller 3 to transmit the control instruction S21 to the display 2 immediately before causing the display 2 to control the color tone or the like of an image. Alternatively, the temperature adjustment control part 11 may cause the transmission part 12 or the remote controller 3 to transmit the control instruction S21 to the display 2 in advance by including an instruction instructing the display 2 to start control at predetermined time in the control instruction S21.

### Second Preferred Embodiment

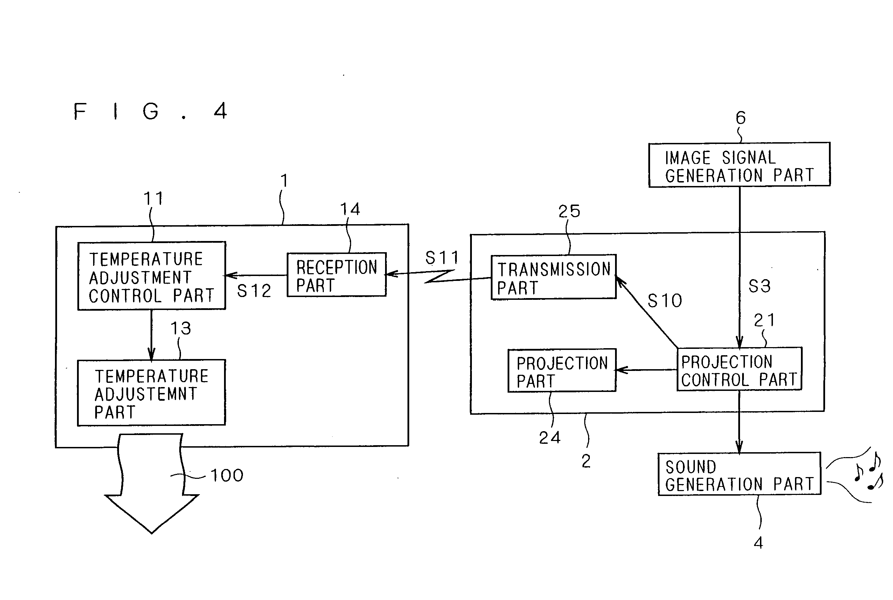

Fig. 4 is a schematic block diagram of an environment adjustment system according to a second preferred embodiment. The system mainly includes the display 2 and the air conditioner 1.

The display 2 includes the projection control part 21, the projection part 24, and a transmission part 25. In Fig. 4, the display 2 is connected to the sound generation part 4 and the image signal generation part 6. While being externally attached to the display 2, the sound generation part 4 and the image signal generation part 6 may be incorporated in the display 2.

The projection control part 21 controls the projection part 24, the transmission part 25, the sound generation part 4, and the image signal generation part 6 as set out below.

The control instruction S10 is input to the transmission part 25, that is caused to transmit the control instruction S11 to the air conditioner 1 based on the control instruction S10. The control instruction S11 causes the air conditioner 1 to control ambient temperature.

The projection part 24, the sound generation part 4, and the image signal generation part 6 are controlled in a similar way to the first preferred embodiment, so the description is omitted. Note that the image displayed or the like by the projection part 24 is subjected to color tone control by the projection control part 21. More specifically, the color tone is controlled to have a calming effect for bedtime, and a stimulating effect for wakeup time.

The air conditioner 1 includes the temperature adjustment control part 11, the temperature adjustment part 13, and the reception part 14.

The reception part 14 receives the control instruction S11 transmitted from the display 2, and inputs the same as the control instruction S12 to the temperature adjustment control part 11.

The temperature adjustment control part 11 controls the temperature adjustment part 13 as set out below. Namely, the temperature adjustment part 13 is caused to adjust ambient temperature based on the control instruction S12, namely, based on the control instruction S11. The temperature adjustment part 13 discharges temperature adjusted air 100 into the environment of a predetermined position, e.g. an environment in which the air conditioner 1 is installed, thereby adjusting the temperature of the environment, namely, ambient temperature.

The control of the color tone of an image in the display 2, and the control of the air conditioner 1 based on the control instruction S11, namely, the control of ambient temperature, are linked with each other. The control technique by the environment adjustment system will now be described for bedtime and for wakeup time independently.

For bedtime, the projection control part 21 first causes the projection part 24 to display or project an image with a color tone having a calming effect in the display 2.

The color tone having a calming effect is obtained by changing the white color temperature to not more than 6000 K. With this color tone, a person looking at the image displayed or the like with this color tone is encouraged to fall asleep.

In addition, the luminance of the image may be decreased in parallel with obtaining the color tone having a calming effect. Accordingly, as the surroundings grow dark, the person looking at the image is further encouraged to fall asleep.

Further, the volume of sound generated by the sound generation part 4 may be decreased in parallel with obtaining the color tone having a calming effect. Accordingly, as the surroundings go quiet, the person listening to the sound is further encouraged to fall asleep.

After the above control, the temperature adjustment control part 11 causes the temperature adjustment part 13 to lower ambient temperature based on the control instruction S 12, namely, based on the control instruction S11, in the air conditioner 1. In this case, it is preferable for the ambient temperature to be lowered from the onset of sleep, so as not to make the person uncomfortable with the feeling of being chilly. The onset of sleep is detected by a sleep sensor, for example.

With such control technique, the person looking at the image feels a color having a calming effect with the change of color tone of the image displayed or the like to one having a calming effect, and is encouraged to fall asleep. And with the subsequent decrease in ambient temperature, the body temperature decreases during sleep. Accordingly, the person is smoothly induced to sleep.

As for wakeup time, the temperature adjustment control part 11 first causes the temperature adjustment part 13 to raise ambient temperature based on the control instruction S12, namely, based on the control instruction S 11, in the air conditioner 1.

Then, the projection control part 21 causes the projection part 24 to display or project an image with a color tone having a stimulating effect in the display 2. In this case, it is preferable for the color tone to have a stimulating effect from the onset of arousal, so as not to make the person uncomfortable with accelerated arousal or the like.
The onset of arousal is detected by an arousal sensor, for example.

The color tone having a stimulating effect is obtained by changing the color temperature to not less than 10000 K. Accordingly, a person looking at the image displayed or the like with this color tone feels less drowsy.

In addition, the luminance of the image may be increased in parallel with obtaining the color tone having a stimulating effect. Accordingly, as the surroundings grow light, the person looking at the image feels less drowsy.

Further, the volume of sound generated by the sound generation part 4 may be increased in parallel with obtaining the color tone having a stimulating effect. Accordingly, as the surroundings get loud, the person listening to the sound feels less drowsy.

With such control technique, the increase in ambient temperature causes the body temperature to increase during sleep, inducing the sleeping person to arousal. And the person looking at the image subsequently displayed or the like after arousal feels a color having a stimulating effect with the change of color tone to one having a stimulating effect, and feels less drowsy. Accordingly, the person is smoothly induced to arousal.

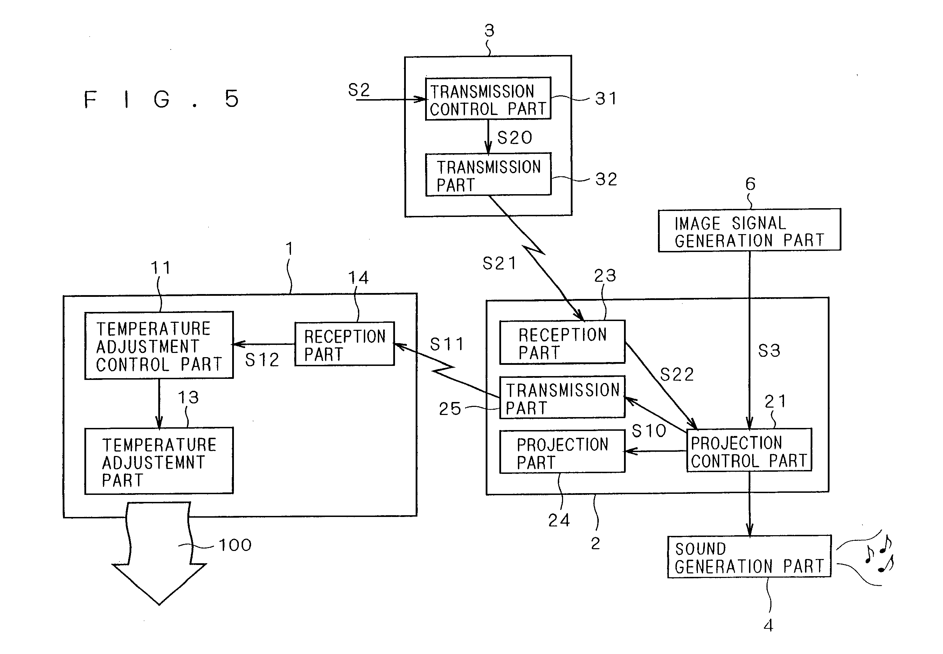

Fig. 5 is a schematic block diagram of the environment adjustment system including the remote controller 3 that communicates with the display 2. In an aspect shown in Fig. 5, the display 2 may control the color tone or the like of an image based on the externally input control instruction S21. The same elements as those of Fig. 4 have the same reference numerals in Fig. 5.

The remote controller 3 includes the transmission control part 31 and the transmission part 32. A control instruction S2 for the display 2 is input to the remote controller 3. The control instruction S2 includes instructions about the color tone of an image, and about starting time of the control.

The transmission control part 31 controls the transmission part 32. More specifically, the control instruction S20 is supplied to the transmission part 32 based on the control instruction S2, and the transmission part 32 is caused to transmit the control instruction S21 to the display 2 based on the control instruction S20. The control instruction S21 causes the display 2 to control the color tone or the like of an image based on the control instruction S2.

The display 2 further includes the reception part 23. The reception part 23 receives the control instruction S21 transmitted from the remote controller 3, and inputs the same as a control instruction S22 to projection control part 21.

The projection control part 21 controls the color tone or the like of an image based on the control instruction S22, namely, based on the control instruction S21. Further, the control instruction S10 is generated based on the control instruction S22, namely, based on the control instruction S21, for causing the transmission part 25 to transmit the control instruction S11. By inputting the control instruction S10, the transmission part 25 is caused to transmit the control instruction S 11 to the air conditioner 1. The control instruction S11 causes the air conditioner 1 to control ambient temperature, as described above.

The air conditioner 1 receives the control instruction S11 by the reception part 14 and, as described above, controls ambient temperature.

Again in this case, the control of the air conditioner 1 based on the control instruction S 11 and the control of the color tone of an image in the display 2 are linked with each other as described above.

With such control technique, only the input of the control instruction S2 for the display 2 to the remote controller 3 allows the air conditioner 1 to be automatically controlled.

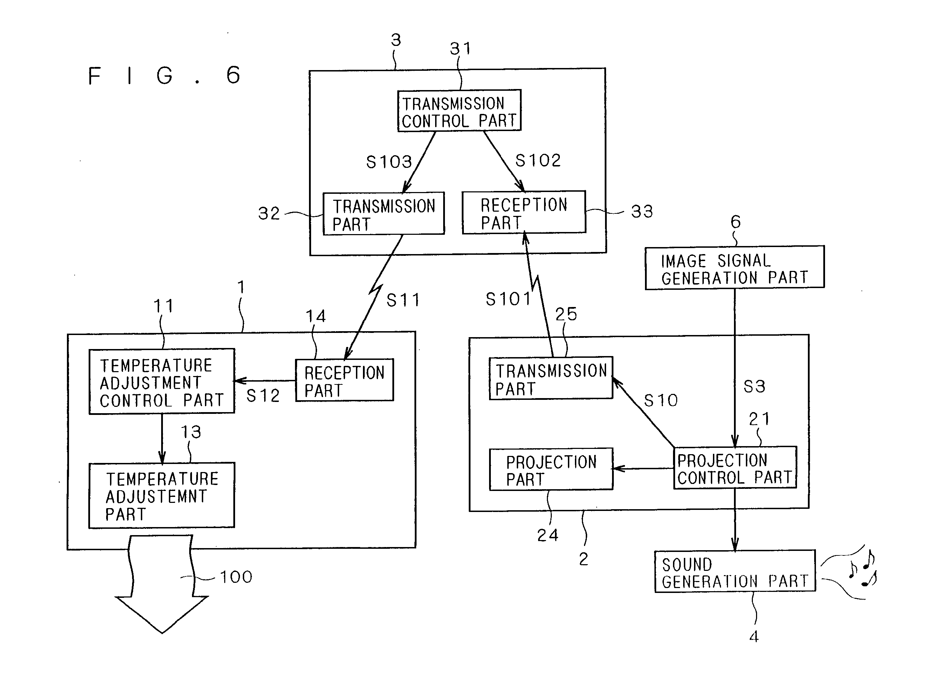

Fig. 6 is a schematic block diagram of another example of the environment adjustment system. In an aspect shown in Fig. 6, the display 2 may cause the air conditioner 1 to control ambient temperature via the remote controller 3. The same elements as those of Fig. 5 have the same reference numerals in Fig. 6.

In the display 2, the projection control part 21 causes the transmission part 25 to transmit a control instruction S101 to the remote controller 3. The control instruction S101 causes the air conditioner 1 to control ambient temperature via the remote controller 3.

The remote controller 3 includes the transmission control part 31, the transmission part 32, and the reception part 33.

The reception part 33 receives the control instruction S101 transmitted from the display 2, and inputs the same as a control instruction S102 to the transmission control part 31.

The transmission control part 31 supplies a control instruction 103 to the transmission part 32 based on the control instruction S102, causing the transmission part 32 to transmit the control instruction S11 to the air conditioner 1.

The air conditioner 1 receives the control instruction S11 by the reception part 14 and, as described above, controls ambient temperature.

Again in this case, the control of the air conditioner 1 based on the control instruction S 11 and the control of the color tone of an image in the display 2 are linked with each other as described above.

With such control technique, the display 2 is capable of automatically controlling the air conditioner 1 via the remote controller 3.

In any of the control techniques described above, the projection control part 21 may cause the transmission part 25 or the remote controller 3 to transmit the control instruction S11 immediately before causing the air conditioner 1 to perform the predetermined control. Alternatively, in advance, the transmission part 25 or the remote controller 3 may be caused to transmit the control instruction S11 which includes an instruction instructing the air conditioner 1 to start control at predetermined time in the control instruction S11, to the air conditioner 1.

### Third Preferred Embodiment

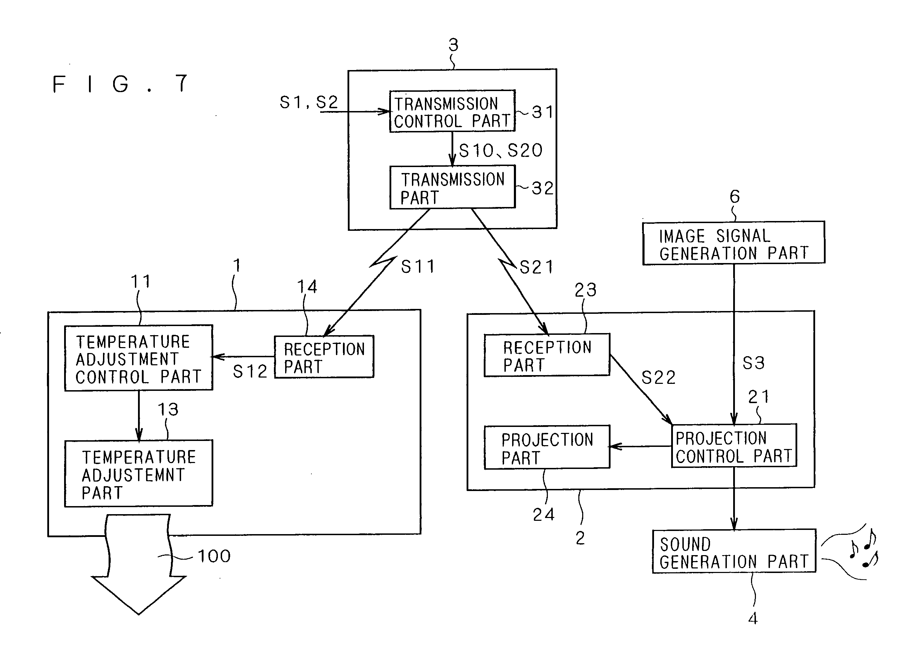

Fig. 7 is a schematic block diagram of an environment adjustment system according to a third preferred embodiment. The system mainly includes the remote controller 3, the air conditioner 1, and the display 2.

The structure of the air conditioner 1 is the same as the air conditioner 1 shown in Fig. 4 that was described in the second preferred embodiment, and the structure of the display 2 is the same as the display 2 shown in Fig. 1 that was described in the first preferred embodiment, so the descriptions are omitted. The same elements as those of Figs. 1 and 4 have the same reference numerals in Fig. 7.

The remote controller 3 includes the transmission control part 31 and the transmission part 32. The control instruction S1 for the air conditioner 1 and the control instruction S2 for the display 2 are respectively input to the remote controller 3. The control instruction S1 includes instructions about ambient temperature to be adjusted by the temperature adjustment part 13. The control instruction S2 includes instructions about the color tone of an image, for example.

The transmission control part 31 controls the transmission part 32. More specifically, the control instruction S10 is supplied to the transmission part 32 based on the control instruction S1, causing the transmission part 32 to transmit the control instruction S11 to the air conditioner 1 based on the control instruction S10. Based on the inputted control instruction S2, the transmission part 32 is supplied with the control instruction S20, based on which the transmission part 32 is caused to transmit the control instruction S11 to the air conditioner 1. The control instruction S11 causes the air conditioner 1 to control ambient temperature based on the control instruction S 1. The control instruction S21 causes the display 2 to control the color tone or the like of an image based on the control instruction S2.

In the air conditioner 1, the reception part 14 receives the control instruction S11, and inputs the same as the control instruction S12 to the temperature adjustment control part 11. The temperature adjustment control part 11 causes the temperature adjustment part 13 to control ambient temperature based on the control instruction S12, namely, based on the control instruction S11. The temperature adjustment part 13 discharges temperature adjusted air 100 into the environment of a prescribed position, e.g. an environment in which the air conditioner 1 is installed, thereby adjusting the temperature of the environment, namely, ambient temperature.

In the display 2, the reception part 23 receives the control instruction S21, and inputs the same as the control instruction S22 to the projection control part 21. The projection control part 21 then controls the color tone of an image based on the control instruction S22, namely, based on the control instruction S21. More specifically, the color tone is controlled to have a calming effect for bedtime, and a stimulating effect for wakeup time.

The control of the air conditioner 1 based on the control instruction S 11 and the control of the display 2 based on the control instruction S21 are linked with each other.

In the environment adjustment system described above, the remote controller 3 is capable of managing the air conditioner 1 and the display 2 in a unified manner. Accordingly, the control of the air conditioner 1 and the control of the display 2 may easily be associated with each other.

The control technique by the environment adjustment system will now be described for bedtime and for wakeup time independently.

For bedtime, the projection control part 21 first causes the projection part 24 to display or project an image with a color tone having a calming effect based on the control instruction S22, namely, based on the control instruction S21, in the display 2.

The color tone having a calming effect is obtained by changing the white color temperature to not more than 6000 K. With this color tone, a person looking at the image displayed or the like with the color tone is encouraged to fall asleep.

In addition, the luminance of the image may be decreased in parallel with obtaining the color tone having a calming effect. Accordingly, as the surroundings grow dark, the person looking at the image is further encouraged to fall asleep.

Further, the volume of sound generated by the sound generation part 4 may be decreased in parallel with obtaining the color tone having a calming effect. Accordingly, as the surroundings go quiet, the person listening to the sound is further encouraged to fall asleep.

After the above control, the temperature adjustment control part 11 causes the temperature adjustment part 13 to lower ambient temperature based on the control instruction S12, namely, based on the control instruction S11, in the air conditioner 1. In this case, it is preferable for the ambient temperature to be lowered from the onset of sleep, so as not to make the person uncomfortable with the feeling of being chilly. The onset of sleep is detected by a sleep sensor, for example.

With such control technique, the person looking at the image feels a color having a calming effect with the change of color tone of the image displayed or the like to one having a calming effect, and is encouraged to fall asleep. And with the subsequent decrease in ambient temperature, the body temperature decreases during sleep. Accordingly, the person is smoothly induced to sleep.

As for wakeup time, the temperature adjustment control part 11 first causes the temperature adjustment part 13 to raise ambient temperature based on the control instruction S12, namely, based on the control instruction S 11, in the air conditioner 1.

Then, the projection control part 21 causes the projection part 24 to display or project an image with a color tone having a stimulating effect based on the control instruction S 12, namely, based on the control instruction S11, in the display 2. In this case, it is preferable for the color tone to have a stimulating effect from the onset of arousal, so as not to make the person uncomfortable with accelerated arousal or the like. The onset of arousal is detected by an arousal sensor, for example.

The color tone having a stimulating effect is obtained by changing the color temperature to not less than 10000 K. Accordingly, a person looking at the image displayed or the like with this color tone feels less drowsy.

In addition, the luminance of the image may be increased in parallel with obtaining the color tone having a stimulating effect. Accordingly, as the surroundings grow light, the person looking at the image feels less drowsy.

Further, the volume of sound generated by the sound generation part 4 may be increased in parallel with obtaining the color tone having a stimulating effect. Accordingly, as the surroundings get loud, the person listening to the sound feels less drowsy.

With such control technique, the increase in ambient temperature causes the body temperature to increase during sleep, inducing the sleeping person to arousal. And the person looking at the image subsequently displayed or the like after arousal feels a color having a stimulating effect with the change of color tone to one having a stimulating effect, and feels less drowsy. Accordingly, the person is smoothly induced to arousal.

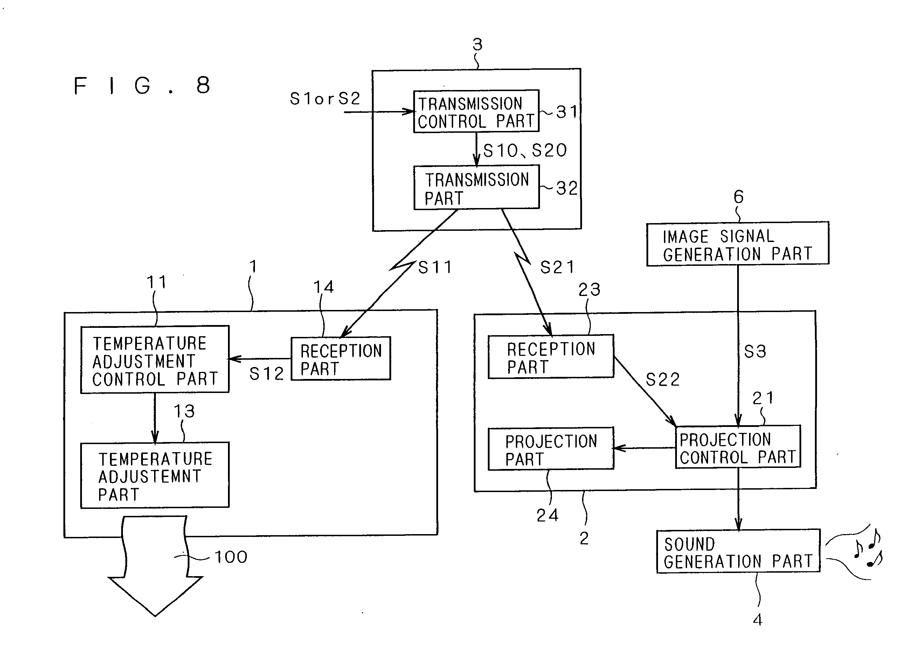

Fig. 8 is a schematic block diagram of another example of the environment adjustment system. The same elements as those of Fig. 7 have the same .reference numerals in Fig. 8.

One of the control instructions S1 and S2 is input to the remote controller 3.

Upon input of the control instruction S1, the transmission control part 31 supplies the control instruction S10 to the transmission part 32, causing the transmission part 32 to transmit the control instruction S 11 based on the control instruction S 1 to the air conditioner 1. Also supplied is the control instruction S20 to the transmission part 32, causing the transmission part 32 to transmit the control instruction S21 based on the control instruction S 1 to the display 2.

With such control technique, only the input of the control instruction S 1 for the air conditioner 1 to the remote controller 3 allows the display 2 to be automatically controlled.

Meanwhile, upon input of the control instruction S2, the transmission control part 31 supplies the control instruction S20 to the transmission part 32, causing the transmission part 32 to transmit the control instruction S21 based on the control instruction S2 to the display 2. Also supplied is the control instruction S10 to the transmission part 32, causing the transmission part 32 to transmit the control instruction S 11 based on the control instruction S2 to the air conditioner 1.

With such control technique, only the input of the control instruction S2 for the display 2 to the remote controller 3 allows the air conditioner 1 to be automatically controlled.

In any of the control techniques described above, the transmission control part 31 may cause the transmission part 12 to transmit the control instructions S11 and S21 to the air conditioner 1 and the display 2, respectively, immediately before causing the air conditioner 1 and the display 2 to perform their respective controls. Alternatively, in advance, the transmission part 12 may be caused to transmit the control instructions S11 and S21 which include instructions instructing the air conditioner 1 and the display 2 to start their respective controls at their prescribed predetermined times in the control instructions S11 and 521, to the air conditioner 1 and the display 2, respectively.

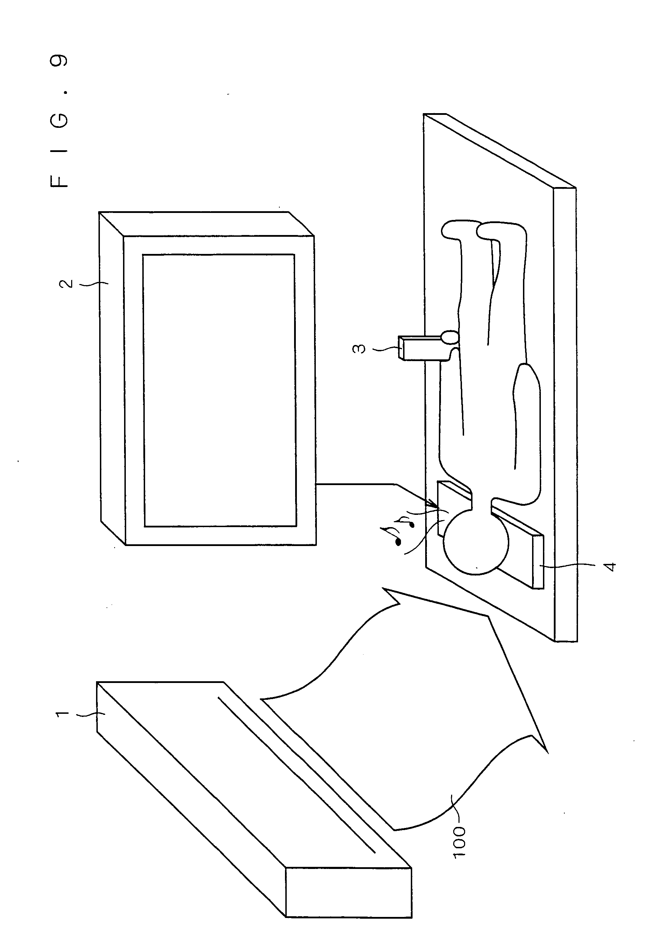

Fig. 9 illustrates a usage pattern of the environment adjustment system. In Fig. 9, the sound generation part 4 is incorporated in a pillow, and the remote controller 3 is illustrated as mobile.

In any of the embodiments described above, the air conditioner 1 incorporating the display 2 may be adopted as the environment adjustment system.

Further, in any of the embodiments described above, a moving image adopted as the image displayed or the like by the display 2 attracts attention. Therefore, the calming effect or stimulating effect by the color tone of the image may be given more efficiently than a still image.

Moreover, in any of the embodiments described above, the turning-on/off of the power to the display 2, particularly to the projection part 24 and the image signal generation part 6, may be controlled by the projection control part 21 or the control instruction S21. For example, the power to the projection part 24 is turned off at the onset of sleep, or turned on at the onset of arousal.

While the invention has been shown and described in detail, the foregoing description is in all aspects illustrative and not restrictive. It is therefore understood that numerous modifications and variations can be devised without departing from the scope of the invention.

## Claims

1. An environment adjustment system comprising:
a projection part (24) for displaying or projecting an image with a variable color tone;
a temperature adjustment part (13) for adjusting ambient temperature; and
a control part (11, 21) for controlling said projection part and said temperature adjustment part respectively, with variations in the color tone of said image and said adjustment to said ambient temperature associated with each other.

2. The environment adjustment system according to claim 1, wherein
said projection part (24) changes said color tone to one having a calming effect, and then said temperature adjustment part (13) lowers said ambient temperature.

3. The environment adjustment system according to claim 2, further comprising
a sleep sensor for detecting the onset of sleep, wherein
said control part (11) controls said temperature adjustment part (13) to lower said ambient temperature from said onset of sleep detected by said sleep sensor.

4. The environment adjustment system according to claim 3, wherein
a color temperature set by said projection part (24) decreases upon changing said color tone to the one having a calming effect.

5. The environment adjustment system according to claim 4, wherein
the luminance of said image decreases upon changing said color tone to the one having a calming effect.

6. The environment adjustment system according to claim 3, wherein
the luminance of said image decreases upon changing said color tone to the one having a calming effect.

7. The environment adjustment system according to claim 2, wherein
a color temperature set by said projection part (24) decreases upon changing said color tone to the one having a calming effect.

8. The environment adjustment system according to claim 7, wherein
the luminance of said image decreases upon changing said color tone to the one having a calming effect.

9. The environment adjustment system according to claim 2, wherein
the luminance of said image decreases upon changing said color tone to the one having a calming effect.

10. The environment adjustment system according to any one of claims 2 through 9, further comprising
a sound generation part (4) for generating sound, wherein
the volume of said sound decreases with the change in said color tone to the one having a calming effect.

11. The environment adjustment system according to claim 10, wherein
said control part includes
a temperature adjustment control part (11) for controlling an operation of said temperature adjustment part (13), and
a projection control part (21) for controlling an operation of said projection part (24),
said environment adjustment system being provided with:
an air conditioner (1) including said temperature adjustment part and said temperature adjustment control part; and
a display (2) including said projection part and said projection control part.

12. The environment adjustment system according to claim 10, wherein
said control part includes
a temperature adjustment control part (11) for controlling an operation of said temperature adjustment part (13),
a projection control part (21) for controlling an operation of said projection part (24), and
a sound control part (21) for controlling an operation of said sound generation part (4),
said environment adjustment system being provided with:
an air conditioner (1) including said temperature adjustment part and said temperature adjustment control part; and
a display (2) including said projection part, said projection control part, and said sound control part.

13. The environment adjustment system according to any one of claims 3 through 9, wherein
said control part includes
a temperature adjustment control part (11) for controlling an operation of said temperature adjustment part (13), and
a projection control part (21) for controlling an operation of said projection part (24),
said environment adjustment system being provided with:
an air conditioner (1) including said temperature adjustment part and said temperature adjustment control part; and
a display (2) including said projection part and said projection control part.

14. The environment adjustment system according to claim 2, wherein
said control part includes
a temperature adjustment control part (11) for controlling an operation of said temperature adjustment part (13), and
a projection control part (21) for controlling an operation of said projection part (24),
said environment adjustment system being provided with:
an air conditioner (1) including said temperature adjustment part and said temperature adjustment control part; and
a display (2) including said projection part and said projection control part.

15. The environment adjustment system according to claim 2, further comprising
a sound generation part (4) capable of generating sound, said sound generation part decreasing the volume of said sound with the change in said color tone to the one having a calming effect, wherein
said control part includes
a temperature adjustment control part (11) for controlling an operation of said temperature adjustment part (13),
a projection control part (21) for controlling an operation of said projection part (24), and
a sound control part (21) for controlling an operation of said sound generation part (4),
said environment adjustment system being provided with:
an air conditioner (1) including said temperature adjustment part and said temperature adjustment control part; and
a display (2) including said projection part, said projection control part, and said sound control part.

16. The environment adjustment system according to claim 1, wherein
said temperature adjustment part (13) raises said ambient temperature, and then said projection part (24) changes said color tone to one having a stimulating effect.

17. The environment adjustment system according to claim 16, further comprising
an arousal sensor for detecting the onset of arousal, wherein
said control part (11) controls said projection part (24) to change said color tone of said image to one having a stimulating effect from said onset of arousal detected by said arousal sensor.

18. The environment adjustment system according to claim 17, wherein
a color temperature set for said projection part (24) increases upon changing said color tone to the one having a stimulating effect.

19. The environment adjustment system according to claim 18, wherein
the luminance of said image increases upon changing said color tone to the one having a stimulating effect.

20. The environment adjustment system according to claim 17, wherein
the luminance of said image increases upon changing said color tone to the one having a stimulating effect.

21. The environment adjustment system according to claim 16, wherein
a color temperature set for said projection part (24) increases upon changing said color tone to the one having a stimulating effect.

22. The environment adjustment system according to claim 21, wherein
the luminance of said image increases upon changing said color tone to the one having a stimulating effect.

23. The environment adjustment system according to claim 16, wherein
the luminance of said image increases upon changing said color tone to the one having a stimulating effect.

24. The environment adjustment system according to any one of claims 16 through 23, further comprising
a sound generation part (4) for generating sound, wherein
the volume of said sound increases with the change in said color tone to the one having a stimulating effect.

25. The environment adjustment system according to claim 24, wherein
said control part includes
a temperature adjustment control part (11) for controlling an operation of said temperature adjustment part (13), and
a projection control part (21) for controlling an operation of said projection part (24),
said environment adjustment system being provided with:
an air conditioner (1) including said temperature adjustment part and said temperature adjustment control part; and
a display (2) including said projection part and said projection control part.

26. The environment adjustment system according to claim 24, wherein
said control part includes
a temperature adjustment control part (11) for controlling an operation of said temperature adjustment part (13),
a projection control part (21) for controlling an operation of said projection part (24), and
a sound control part (21) for controlling an operation of said sound generation part (4),
said environment adjustment system being provided with:
an air conditioner (1) including said temperature adjustment part and said temperature adjustment control part; and
a display (2) including said projection part, said projection control part, and said sound control part.

27. The environment adjustment system according to any one of claims 17 through 23, wherein
said control part includes
a temperature adjustment control part (11) for controlling an operation of said temperature adjustment part (13), and
a projection control part (21) for controlling an operation of said projection part (24),
said environment adjustment system being provided with:
an air conditioner (1) including said temperature adjustment part and said temperature adjustment control part; and
a display (2) including said projection part and said projection control part.

28. The environment adjustment system according to claim 16, wherein
said control part includes
a temperature adjustment control part (11) for controlling an operation of said temperature adjustment part (13), and
a projection control part (21) for controlling an operation of said projection part (24),
said environment adjustment system being provided with:
an air conditioner (1) including said temperature adjustment part and said temperature adjustment control part; and
a display (2) including said projection part and said projection control part.

29. The environment adjustment system according to claim 16, further comprising
a sound generation part (4) capable of generating sound, said sound generation part increasing the volume of said sound with the change in said color tone to the one having a stimulating effect, wherein
said control part includes
a temperature adjustment control part (11) for controlling an operation of said temperature adjustment part (13),
a projection control part (21) for controlling an operation of said projection part (24), and
a sound control part (21) for controlling an operation of said sound generation part (4),
said environment adjustment system being provided with:
an air conditioner (1) including said temperature adjustment part and said temperature adjustment control part; and
a display (2) including said projection part, said projection control part, and said sound control part.

30. The environment adjustment system according to any one of claims 14, 15, 28 and 29, wherein
said air conditioner (1) further includes a transmission part (12) for transmitting a first control instruction (S21),
said display (2) further includes a first reception part (23) for receiving said first control instruction,
said temperature adjustment control part (11) causes said transmission part to transmit said first control instruction to said first reception part, and
said projection control part (21) causes said projection part to display or project said image with a change in said color tone based on said first control instruction received by said first reception part.

31. The environment adjustment system according to claim 30, further comprising
a remote controller (3) for transmitting a second control instruction (S11), wherein
said air conditioner (1) further includes a second reception part (14) for receiving said second control instruction, and
said temperature adjustment control part (11) causes said temperature adjustment part (13) to control said ambient temperature based on said second control instruction received by said second reception part, and causes said transmission part (12) to transmit said first control instruction (S21) to said first reception part (23) based on said second control instruction.

32. The environment adjustment system according to any one of claims 14, 15, 28 and 29, further comprising
a remote controller (3) for transmitting a first control instruction (S21), wherein
said air conditioner (1) further includes a transmission part (12), said transmission part transmitting a second control instruction (S201),
said display (2) further includes a first reception part (23) for receiving said first control instruction,
said temperature adjustment control part (11) causes said transmission part (12) to transmit said second control instruction to said remote controller,
said remote controller transmits said first control instruction to said first reception part based on said second control instruction, and
said projection control part (21) causes said projection part to display or project said image with a change in said color tone based on said first control instruction received by said first reception part.

33. The environment adjustment system according to any one of claims 14,15, 28 and 29, further comprising
a remote controller (3), said remote controller including:
a transmission part (32) for transmitting a first control instruction (S21) instructing control of said projection part (24) and a second control instruction (S11) instructing control of said temperature adjustment part (13); and
a transmission control part (31) for controlling said transmission part, wherein
said display (2) further includes a first reception part (23) for receiving said first control instruction,
said air conditioner (1) further includes a second reception part (14) for receiving said second control instruction,
said transmission control part causes said transmission part to transmit said first control instruction to said first reception part and said second control instruction to said second reception part,
said projection control part (21) causes said projection part (24) to display or project said-image with a change in said color tone based on said first control instruction received by said first reception part, and
said temperature adjustment control part (11) causes said temperature adjustment part (13) to control said ambient temperature based on said second control instruction received by said second reception part.

34. The environment adjustment system according to claim 33, wherein
a third control instruction (S1) instructing control of said air conditioner (1) is input to said transmission control part (31), and
said transmission control part causes said transmission part (32) to transmit said second control instruction (S11) based on said third control instruction to said second reception part (14), and causes said transmission part to transmit said first control instruction (S21) based on said third control instruction to said first reception part (23).

35. The environment adjustment system according to claim 33, wherein
a third control instruction (S2) instructing control of said display (2) is input to said transmission control part (31), and
said transmission control part causes said transmission part (32) to transmit said first control instruction (S21) based on said third control instruction to said first reception part (23), and causes said transmission part to transmit said second control instruction (S11) based on said third control instruction to said second reception part (14).

36. An environment adjustment method comprising:
a projection step of changing a color tone of an image displayed or projected on a display (2) to one having a calming effect; and
a temperature adjustment step of lowering ambient temperature after said projection step.

37. The environment adjustment method according to claim 36, wherein
said ambient temperature is lowered from the onset of sleep in said temperature adjustment step.

38. The environment adjustment method according to claim 37, wherein
a color temperature of said color tone is lowered upon changing said color tone to the one having a calming effect in said projection step.

39. The environment adjustment method according to claim 38, wherein
the luminance of said image is decreased upon changing said color tone to the one having a calming effect in said projection step.

40. The environment adjustment method according to claim 37, wherein
the luminance of said image is decreased upon changing said color tone to the one having a calming effect in said projection step.

41. The environment adjustment method according to claim 36, wherein
a color temperature of said color tone is lowered upon changing said color tone to the one having a calming effect in said projection step.

42. The environment adjustment method according to claim 41, wherein
the luminance of said image is decreased upon changing said color tone to the one having a calming effect in said projection step.

43. The environment adjustment method according to claim 36, wherein
the luminance of said image is decreased upon changing said color tone to the one having a calming effect in said projection step.

44. The environment adjustment method according to any one of claims 36 through 43, wherein
a sound generation part (4) generates sound in parallel with the display or projection of said image on said display (2), and
the volume of said sound is decreased with the change in said color tone to the one having a calming effect in said projection step.

45. An environment adjustment method comprising:
a temperature adjustment step of raising ambient temperature; and
a projection step of changing a color tone of an image displayed or projected on a display (2) to one having a stimulating effect after said temperature adjustment step.

46. The environment adjustment method according to claim 45, wherein
said color tone is changed to the one having a simulating effect from the onset of arousal in said projection step.

47. The environment adjustment method according to claim 46, wherein a color temperature of said color tone is raised upon changing said color tone to the one having a stimulating effect in said projection step.

48. The environment adjustment method according to claim 47, wherein
the luminance of said image is increased upon changing said color tone to the one having a stimulating effect in said projection step.

49. The environment adjustment method according to claim 46, wherein
the luminance of said image is increased upon changing said color tone to the one having a stimulating effect in said projection step.

50. The environment adjustment method according to claim 45, wherein
a color temperature of said color tone is raised upon changing said color tone to the one having a stimulating effect in said projection step.

51. The environment adjustment method according to claim 50, wherein
the luminance of said image is increased upon changing said color tone to the one having a stimulating effect in said projection step.

52. The environment adjustment method according to claim 45, wherein
the luminance of said image is increased upon changing said color tone to the one having a stimulating effect in said projection step.

53. The environment adjustment method according to any one of claims 45 through 52, wherein
a sound generation part (4) generates sound in parallel with the display or projection of said image on said display (2), and
the volume of said sound is increased with the change in said color tone to the one having a stimulating effect in said projection step.

54. An environment adjustment method for an environment adjustment system, said system comprising:
an air conditioner (1) for executing said temperature adjustment step included in the environment adjustment method according to any one of claims 36 through 43 and claims 45 through 52; and
a display (2) for executing said projection step according to any one of said claims,
said air conditioner including a transmission part (12) for transmitting a first control instruction (S21), and
said display including a first reception part (23) for receiving said first control instruction,
said method comprising:
a transmission step of causing said transmission part to transmit said first control instruction to said first reception part; and
a step of executing said projection step based on said first control instruction after receiving said first control instruction by said first reception part.

55. The environment adjustment method according to claim 54 for said environment adjustment system, said system further comprising
a remote controller (3) for transmitting a second control instruction (S11),
said air conditioner (1) further including a second reception part (14) for receiving said second control instruction,
said method executing, before said transmission step,:
a step of causing said remote controller to transmit said second control instruction; and
a step of executing said temperature adjustment step based on said second control instruction after receiving said second control instruction by said second reception part, and generating said first control instruction (S21) based on said second control instruction in said air conditioner (1).

56. An environment adjustment method for an environment adjustment system, said system comprising:
an air conditioner (1) for executing said temperature adjustment step included in the environment adjustment method according to any one of claims 36 through 43 and claims 45 through 52;
a display (2) for executing said projection step according to any one of said claims; and
a remote controller (3) for transmitting a first control instruction (S21),
said air conditioner (1) including a transmission part (12), said transmission part transmitting a second control instruction (S201), and
said display (2) including a first reception part (23), said first reception part receiving said first control instruction,
said method comprising:
a step of causing said transmission part to transmit said second control instruction to said remote controller;
a step of causing said remote controller having received said second control instruction to transmit said first control instruction to said first reception part based on said second control instruction; and
a step of executing said projection step based on said first control instruction after receiving said first control instruction by said first reception part.

57. An environment adjustment method for an environment adjustment system, said system comprising:
an air conditioner (1) for executing said temperature adjustment step included in the environment adjustment method according to any one of claims 36 through 43 and claims 45 through 52;
a display (2) for executing said projection step according to any one of said claims; and
a remote controller (3),
said display (2) further including a first reception part (23), said first reception part receiving a first control instruction (S21),
said air conditioner (1) further including a second reception part (14) for receiving a second control instruction (S 11), and
said remote controller including a transmission part (32) for transmitting said first and second control instructions,
said method comprising the steps of:
(a) causing said transmission part to transmit said first control instruction (S21) to said first reception part (23) and said second control instruction (S11) to said second reception part (14);
(b) executing said projection step based on said first control instruction after receiving said first control instruction by said first reception part; and
(c) executing said temperature adjustment step based on said second control instruction after receiving said second control instruction by said second reception part.

58. An environment adjustment method executing, before said step (a),
a step of inputting a third control instruction (S1) instructing execution of said temperature adjustment step to said remote controller (3),
wherein in said step (a),
said transmission part (32) is caused to transmit said second control instruction (S11) based on said third control instruction to said second reception part (14), and
said transmission part is caused to transmit said first control instruction (S21) based on said third control instruction to said first reception part (23).

59. An environment adjustment method executing, before said step (a),
a step of inputting a third control instruction (S2) instructing execution of said projection step to said remote controller (3),
wherein in said step (a),
said transmission part (32) is caused to transmit said first control instruction (S21) based on said third control instruction to said first reception part (23), and
said transmission part is caused to transmit said second control instruction (S 11) based on said third control instruction to said second reception part (14).

60. An air conditioner comprising:
a transmission part (12) for transmitting a first control instruction (S21) to a display (2) for displaying or projecting an image with a variable color tone;
a temperature adjustment part (13) for adjusting ambient temperature; and
a temperature adjustment control part (11) for controlling said temperature adjustment part,
said temperature adjustment control part causing said transmission part to transmit said first control instruction to said display, and
said first control instruction causing said display to control said color tone of said image in association with said temperature adjustment control part causing said temperature adjustment part to adjust said ambient temperature.

61. A remote controller comprising:
a transmission part (32); and
a transmission control part (31) for controlling said transmission part,
said transmission part being capable of transmitting a first control instruction (S21) to a display (2) for displaying or projecting an image with a variable color tone, and of transmitting a second control instruction (S 11) to an air conditioner (1),
said transmission control part causing said transmission part to transmit said first control instruction (S21) to said display and said second control instruction (S11) to said air conditioner,
said first control instruction causing said display to control a color tone of said image,
said second control instruction causing said air conditioner to adjust ambient temperature, and
variations in said color tone of said image and said adjustment to said ambient temperature are associated with each other.
